Europäisches Patentamt

European Patent Office

Office européen des brevets

(11) Publication number: **0 336 413 B1**

## EUROPEAN PATENT SPECIFICATION

(45) Date of publication of patent specification: **15.06.94**

(51) Int. Cl.5: **C12N 15/74**, C12N 1/21,
//(C12N1/21,C12R1:19),
(C12N1/21,C12R1:07)

(21) Application number: **89106060.0**

(22) Date of filing: **06.04.89**

(54) **A novel system of inducible positive regulation of use for production of heterologous proteins.**

(30) Priority: **08.04.88 IT 2013388**

(43) Date of publication of application:
**11.10.89 Bulletin 89/41**

(45) Publication of the grant of the patent:
**15.06.94 Bulletin 94/24**

(84) Designated Contracting States:
**AT BE CH DE ES FR GB GR IT LI LU NL SE**

(56) References cited:
**EP-A- 0 232 229**

**JOURNAL OF BACTERIOLOGY, vol. 170, no. 7, July 1988, Washington DC, S. STIBITZ et al. "Genetic Analysis of a Region of the Bordetella pertussis Chromosome Encoding Filamentous Hemagglutinin and the pleiotropic Regulatory Locus vir", pp. 2904-2913**

**JOURNAL OF BACTERIOLOGY, vol. 169, no. 6, June 1987, Washington DC, A. NICOSIA et al. "Promoter of the Pertussis Toxin Operon and Production of Pertussis Toxin", pp.**

2843-2846

(73) Proprietor: **SCLAVO S.p.A.**
**Via Fiorentina 1**
**I-53100 Siena(IT)**

(72) Inventor: **Gross, Roy**
**Strada di Pieve al Bozzone, 34**
**I-53100 Siena(IT)**
Inventor: **Rappuoli, Rino**
**Via Calamandrei, 35**
**I-53010 Ouercegrossa (Siena)(IT)**

(74) Representative: **Hallybone, Huw George et al**
**CARPMAELS AND RANSFORD**
**43 Bloomsbury Square**
**London WC1A 2RA (GB)**

EP 0 336 413 B1

**Description**

The invention relates to a novel system of inducible positive regulation capable of controlling large-scale production of polypeptides by microbial expression of heterologous genes. The invention also relates to recombinant vectors comprising the regulating system or components thereof, and microorganisms transformed by the vectors.

As a result of recent developments in genetic engineering it has become possible to produce known or novel molecules of use in therapy, food or agriculture, by methods of fermentation using microorganisms transformed by recombinant DNA techniques.

These techniques usually comprise:

(a) Isolating the DNA gene or fragment comprising the gene which codes for the protein of interest;

(b) Inserting the gene into a cloning vector and isolating the recombinant vector capable of expressing the cloned gene;

(c) Transforming a host microorganism by means of the recombinant vector, and finally

(d) Cultivating the transformed microorganism in a suitable environment for producing the polypeptide coded by the cloned gene.

One important factor in applying the aforementioned technique is the cloning vector, which can be chosen from among plasmids, phages or cosmids. Preferably use is made of the plasmid, a circular DNA molecule capable of replicating independently and advantageously containing the genes for resistance to antibiotics and facilitating recognition of cells transformed with the aforementioned plasmids. These cells also grow in selective media containing antibiotics.

Also, plasmids can be cut in specific manner by various restriction enzymes, each of which recognizes a different site in the plasmid DNA.

Heterologous genes or DNA fragments containing these genes are therefore inserted into the plasmids in the cutting site.

The term "heterologous" refers to a gene normally not present in the plasmid, or a polypeptide normally not produced by a microorganism transformed thereby.

The resulting recombinant or hybrid plasmids can then be introduced into a host microorganism by the techniques of transformation or conjugation.

When the gene is inserted into the plasmid in the proper manner with reference to the region which controls transcription and translation thereof, the resulting hybrid plasmid can be used to produce or express the polypeptide coded by the gene.

"Transcription" is defined as the mechanism via which the genetic information is transmitted from DNA to messenger RNA (mRNA) whereas "translation" means the information whereby genetic information passes from the mRNA to the protein in accordance with the rules of the genetic code.

Accordingly the term "expression" denotes the method whereby genetic information contained in DNA is used for producing the protein.

Transcription is brought about by the DNA-dependent RNA polymerase enzyme present in the cells, and begins in a region known as the promoter and containing a recognition site and an enzyme attachment site.

The RNA polymerase leaving the attachment site and progressively opening the double helix, moves in the $3' \rightarrow 5'$ direction of the matrix helix, whereas polymerization of the ribonucleotides continues in the $5' \rightarrow 3'$ direction. While the polymerase is moving forward, the transcribed DNA returns to the double helix structure.

The resulting molecule of RNA (mRNA), which comprises the coding nucleotides for the ribosome attachment site (RBS) and the initial protein translation sequence ATG, is then bonded by the ribosomes.

The information in the mRNA is thus used by the cell in order to synthesize the protein.

The expressed protein is then recovered from the transformed cells or from the culture medium and is in the form of a mature protein or a fused protein, i.e. comprising the amino-acid sequence of the protein of interest and also comprising a foreign sequence at the amine terminal thereof.

Genetic engineering can play an important part in the industrial production of proteins of various origin by means of transformed bacterial cells.

This is the case for example with human insulin, interferon, the growth hormone and products used for development of vaccines such as the pertussis toxin.

However, methods using microorganisms transformed by recombinant DNA techniques have limitations due mainly to the low rates of production, as a result of which the method is of little industrial interest.

This may be due to inefficiency of the promoter or to premature destruction of the microbial cells in the case where the expressed polypeptides are lethal or harmful to the microorganism. In the art, therefore, regulating systems have been proposed for use in the construction of recombinant DNA molecules so as to improve the expression of genes, so that the polypeptides coded by the genes can be produced in high yields.

As is known, genetic regulating mechanisms can act in a negative or a positive sense.

At present very little is known about the positive regulating mechanism and the control systems used by the cells, whereas there are numerous known systems of negative regulation, some of which have been used to construct recombinant plasmids by expression of heterologous proteins.

The known systems, however, have disadvantages due to the presence of weak promoters or the need to use high levels of inductor in the culture medium.

There has now been found a novel system of positive regulation which can easily be induced by suitable selection of the culture medium and the growth conditions, and is of use for producing high yields of heterologous proteins.

One aim of the invention therefore is a novel system of inducible positive regulation of use for constructing a vector which can be replicated by expression and can be used in the production of heterologous proteins via transformed microorganisms.

In the vectors which can be replicated by expression, the regulating system or regions thereof are suitably bonded to a heterologous gene which codes for a protein of interest.

The invention also relates to vectors which can be replicated by expression and comprise the regulating system, and microorganisms transformed by the vectors.

The invention also relates to a method for controllable production of a polypeptide of interest in high yields, by using microorganisms which use the aforementioned system. The invention also relates to use of the regulating system and/or constituents thereof for constructing vectors which can be replicated by expression for producing heterologous proteins.

According to the invention, the inducible positive regulation system comprises the vir region, a region of about 4.5 kb situated between the EcoRI and XhoI site of the Bordetella chromosome containing the genes BvgA, BvgB and BvgC which code for the regulating factors, and a terminal 5′ region situated in front of a gene which codes for a Bordetella protein capable of being activated or repressed in trans by the aforementioned regulating factors.

More particularly, the terminal 5′ regions transactivated by the regulating factor and/or factors are those situated in front of a gene (i) which codes for one of the virulent proteins produced by Bordetella.

More particularly when the terminal 5′ region is the sequence situated upstream of the promoter of the coding operon the pertussis toxin (PT) said Bordetella is Bordetella pertussis.

The terminal regions capable of being inhibited by the regulation factors are the sequences situated upstream of a gene which codes a non-virulent protein.

For example the non-coding terminal 5′ region contained in the chromosomal DNA fragment of Bordetella comprising the coding nucleotide sequence for the aminoterminal part of FHA (see example 7).

According to the invention the vir region of Bordetella, hereinafter called the regulating region, is completely induced by using temperatures of 35°C or thereabouts and/or a culture medium free from modulating substances such as $MgSO_4$, NaCl or nicotinic acid.

The term "complete induction" means the capacity of the aforementioned region to produce the regulating factor and/or factors which, by transactivating a terminal 5′ region capable of being activated, induce production of the heterologous proteins.

According to the invention the regulating region can easily be repressed by using a culture medium also containing the aforementioned modulating substances and/or at ambient temperatures (20 - 25°C).

Under these conditions the system is in the inactivated form and does not produce the regulating factor and/or factors and consequently the terminal 5′ regions which were inhibited by these factors can now enable the promoter positioned immediately downstream; to guide the expression of the gene under its control.

According to the invention, the vir region is advantageously capable of being induced or repressed either when present as a single copy in the genome of a host organism or when incorporated in a number of copies as an extra chromosomal element.

The regulating system according to the invention is therefore particularly useful for controlled production of high yields of polypeptides by using microorganisms transformed by recombinant vectors using the present system.

More particularly the recombinant vectors can be constructed by suitably inserting a terminal 5′ region or fragments thereof, either alone or in combination with the DNA fragment comprising the vir region, in a cloning vector chosen from among those generally used in the art.

Preferably, when host microorganisms selected from the Bordetella group are used, the recombinant vector used comprises the terminal 5′ region only.

In an embodiment of the invention, therefore, recombinant vectors can be prepared by suitably introducing a terminal 5′ region into a cloning vector, the region being capable of being transactivated by the regulating factor and/or factors in combination or not in combination with the vir region, and by positioning downstream of the region a gene which codes for the protein of interest. In that case the heterologous protein will be expressed by cultivating microorganisms transformed by the vectors in the absence of modulators in the culture medium and at temperatures of 35°C or thereabouts.

According to another embodiment of the invention, recombinant vectors can be constructed by using a terminal 5′ region capable of being inhibited by the regulation factor and/or factors in combination or not in combination with the sequence containing the Bordetella vir region.

In that case the heterologous gene positioned downstream of the terminal 5′ region will be expressed by cultivating microorganisms transformed by the vector in the presence of the modulating compounds and at ambient temperatures (20 - 25°C).

The method used for constructing the vectors by expression consists in cloning the system in a vector and positioning a coding gene for the desired protein downstream of the terminal 5′ region.

According to the invention the heterologous gene can be placed under the control of homologous or heterologous promoters not subject to regulation and made responsive by incorporating therein the regulating sequences according to the invention.

The resulting recombinant vectors are then used to transform a host microorganism.

Microorganisms used for expressing heterologous proteins by the system according to the invention are chosen from among E. coli, B. subtilis and Bordetella. The preferred embodiment of the invention is described with reference to E.coli and Bordetella.

The invention also includes detection and description of the terminal 5′ region of the promoter region situated upstream of the promoter of the operon of the pertussis (PT) toxin of B.pertussis capable of being transactivated by the vir regulating region and capable of simulating transcription of the structural gene inserted downstream of the last-mentioned region.

The pertussis toxin (PT) is a protein made up of five different sub-units which, in the virulence phase or phase I, are produced only by Bordetella pertussis, a bacterium which causes pertussis in man.

The other two species, B. parapertussis and B. bronchiseptica, comprise the coding genes for a functionally active PT but are unable to express them (B. Aricò and R. Rappuoli, (1987) J. Bacteriol. 169, 2847-2853).

The co-pending Italian patent Application A/86 No. 19208 describes and claims the cloning and sequencing of the coding gene or genes for PT of B. pertussis and shows that the coding genes for the individual sub-units of PT are combined in a single operon.

As a result of analysis of the sequence of the aforementioned operon , the promoter has also been defined as the region about 40 bp away from the initial transcription site and comprising the consensus sequences -35 and -10 closely resembling the sequences of the promoters of E.coli.

It has been found however that cells of E. coli transformed by plasmid PT101 comprising the PT operon produce the pertussis toxin in extremely low quantities. Said plasmid PT101 was deposited on October 10, 1988 at the American Type Culture Collection and was allotted the deposit number ATCC 67854.

This indicated that the promoter defined by us, though possessing the sequences recognized by DNA-dependent polymerase RNA, was inefficient in the case of this microorganism.

According to the invention, and for the purpose of defining the region or regions necessary for expression of PT, the DNA EcoRI-HindIII 539 bp fragment comprising the PT promoter and its terminal 5′ region was isolated from plasmid PT101 ATCC 67854. Deletion mutants were then prepared by partially digesting the fragment with Ba131 exonuclease (R. J. Legerski et al (1978), Nucl. Acids Res. 5, 1445) so as to remove certain sequences from the region upstream of the PT promoter.

In practice the purified fragment was introduced by generally known methods into a cloning vector previously digested by suitable restriction enzymes.

Preferably use was made of the bluescript SK vector (commercially available, Stratagene, CA, USA) digested with EcoRI and HindIII restriction enzymes.

The resulting plasmid was then linearized at its EcoRI site in buffer solution and portions of the solution were partially digested with Ba131. After the digestion mixtures had been treated with the Klenow fragment of polymerase DNA to flatten the terminals, the DNA was cut with HindIII.

The resulting restriction fragments were separated from the gel by known methods and those containing from 50 to 250 bases were electro-eluted and re-cloned in a vector for analyzing the sequence.

DNA fragments containing the following deletions were identified in this way:

| Deleted fragments | Promoter PT | Deleted region |
|---|---|---|
| Δ 2 | - 483 - + 1 | None |
| Δ 14 | - 183 - + 1 | 5' 300 bp |
| Δ 25 | - 171 - + 1 | 5' 312 bp |
| Δ 20 | - 158 - + 1 | 5' 325 bp |
| Δ 24 | - 149 - + 1 | 5' 334 bp |
| Δ 46 | - 130 - + 1 | 5' 353 bp |
| Δ 28 | - 123 - + 1 | 5' 360 bp |
| Δ 72 | - 97 - + 1 | 5' 386 bp |
| Δ 33 | - 80 - + 1 | 5' 403 bp |
| Δ 1 | - 62 - + 1 | 5' 421 bp |
| Δ 22 | - 37 - + 1 | 5' 446 bp |
| In this Table, + 1 indicates the transcription recognition site. | | |

The BamHI-HindIII fragments were then isolated and introduced into a cloning vector chosen from among those known in the art, by inserting a heterologous gene downstream of the fragments, the gene being without a promoter of its own.

According to the invention, was was made of the vector pLAFR2 (A. M. Friedman et al. (1982), Gene 18, 289-296) and the coding gene for chloramphenicol acetyl transferase (CAT).

The resulting recombinant plasmids, denoted by pBP ( 1, 2, 14, 20, 22, 24, 25, 28, 33, 46 and 72) comprising the aforementioned deletions were isolated and used to conjugate various species of Bordetella.

More particularly, use was made of B. pertussis BP 356 (ptx:Tn 5) vir$^+$ and B. pertussis BP 347 (Vir: Tn 5) vir$^-$ (A.A.Weiss and S.Falkow, (1983) Infect. Immun. 42, 33-41), available at any research laboratory.

The aforementioned strains were transformed by conjugation, using as the donator strain E. coli SM10 - (available at laboratories) transformed with the aforementioned recombinant plasmids and following the technique described by J. Bordet and O. Gengou (1909) (Amm. Inst. Pasteur (Paris) 23, 415-419).

The resulting ex-conjugates were then cultivated in a suitable medium, preferably Stainer - Scholte medium (D. W. Stainer and M. J. Scholte (1971) J. Gen. Microbiol. 63, 211-220) for the period necessary for obtaining the maximum concentration of cells.

The cells were lysed by known general methods, followed by qualitative and quantitative determination of the CAT expressed by the aforementioned transformed strains, by analysis of its activity (C. M. Gorman et al (1982), Mol. Cell. Biol. 2, 1044-1051; A. Nicosia and R. Rappuoli (1987), J. Bacteriol 169, 2843-2846).

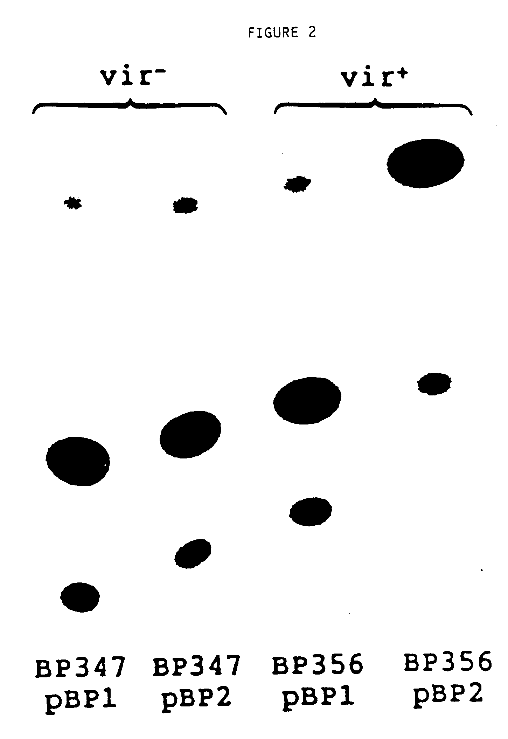

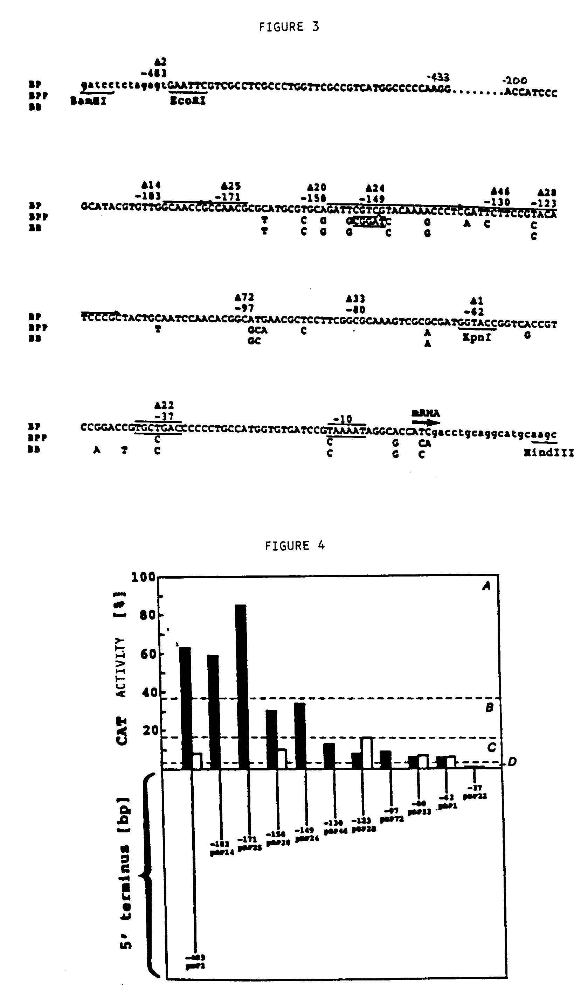

The results, given in Figs. 2 and 4, show that large-scale expression of CAT was observed only in BP356 (vir$^+$) strains containing plasmids in which the region upstream of the PT promotor contained at least 170 bp.

The absence of this region, as in the case of hybrid pBP1 plasmid or an active vir region as in Bordetella BP 347, resulted in a considerable decrease in expression of CAT.

This indicated that at least two conditions were required for efficient transcription of the heterologous gene:

1. The presence of a terminal 5' region of at least 170 bp upstream of the initial transcription site and
2. A transactivating factor (i) coded by the vir region.

A 45% reduction in CAT activity was in fact observed when the sequence between -70 and -158 was deleted.

A further decrease in CAT activity, about 10% of original activity, was found when the region between -149 and -130 was removed.

Finally the activity decreased to values below 1% when the deletion destroyed the -35 sequence of the promoter.

Analysis of the resulting sequence of the terminal 5' region also showed that the region contained structures typical of the attachment sites of regulating proteins described in the case of other genes (T. Mizuno and S. Mizushimo (1986) J. Bacteriol, 168, 86-95, O. Raibaud and M. Schwartz (1984) Ann. Rev., Genet., 18, 173-206).

More particularly the following were detected:

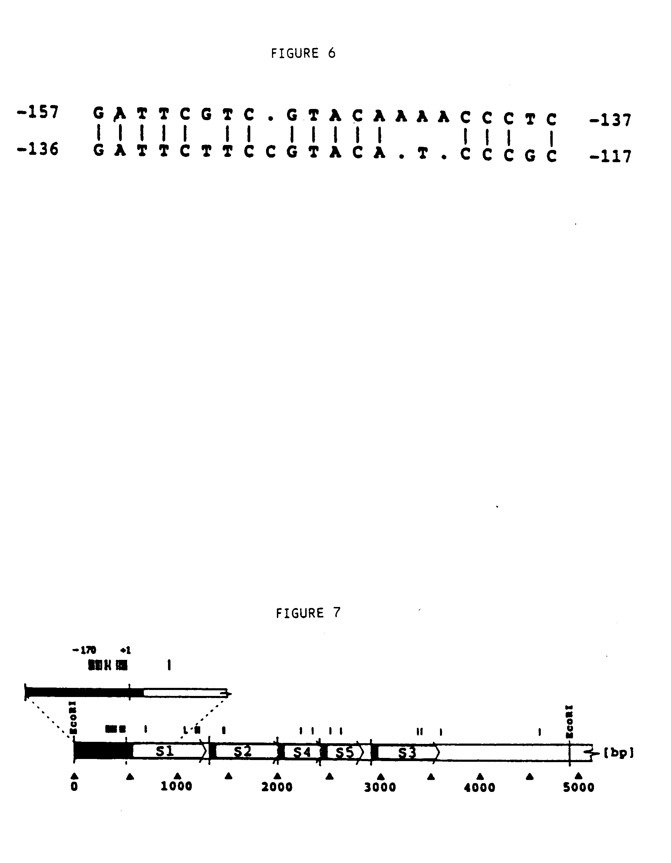

- a palindromic sequence contained in the region from -182 to -170 (Fig. 3) and
- a 21 bp sequence in the position from -157 to -137, repeated after two turns of the DNA helix (Figs. 3 and 6).

The results can be used to correlate the CAT activity for the various deletion mutants having the structural properties of the DNA sequence, and they can be grouped in four levels (A, B, C and D) (Fig. 4).

Level A: When at least 170 bp of the terminal 5′ region of the PT promoter are present, activity is at a maximum (pBP2, pBP25, pBP14);

Level B: Removal of part of the first repeated sequence (pBP 24) or of the region immediately upstream of it (pBP 20) reduces the CAT activity by 45%.

Level C: When the second repeated sequence is partially or completely removed (pBP46, pBP28, pBP72, pBP33 and pBP1), the terminal 5′ region loses the capacity to be transactivated.

This indicates that the repeated sequence (Fig. 6) is very probably the attachment site of the transactivating factor (i).

Level D: When the -35 sequence of the promoter is removed (pBP22) the CAT activity decreases to values below 1% in both the vir$^+$ and vir$^-$ strains. It is also noteworthy that removal of the sequence from -182 to -171 containing the palindromic sequence resulted in a significant increase in CAT activity. This suggested that this sequence very probably plays a critical part in regulating the transcription of heterologous genes.

According to the invention and for the purpose of checking the results, two plasmids were constructed. One, plasmid pLAFR-CAT, was without the PT promoter and the terminal 5′ region thereof and in the other, pBP255, the region upstream of the Kpn1 (-62) restriction site had been replaced by the 600 bp BamHi-HindIII fragment which codes for the S1 sub-unit of PT without a promoter.

No CAT activity was observed in Bordetella vir$^+$ and vir$^-$ transformed by plasmid pLAFR-CAT, whereas vir$^+$ cells containing plasmid pBP255 showed the same activity as observed in the case of the pBP1 deletion mutant.

These results confirmed that the specific sequences upstream of the PT promoter were necessary for transactivation thereof.

According to the invention it has been found that the aforementioned recombinant plasmids, when introduced into virulent B. parapertussis and B. bronchiseptica, enabled the microorganisms to express the CAT gene in quantities identical with those measured in the case of B. pertussis BP357 (vir$^+$).

This indicated that efficient vir regions were present in the aforementioned bacteria and could be used for the purposes of the invention.

According to the invention, a study was made of the effect of mutations present in the terminal 5′ region on the activity of the promoter and on the efficiency of transcription thereof.

To this end, the region between the EcoRI site (-483) and the KpnI site (-62) of plasmid pBP2 was replaced by the corresponding region of B. bronchiseptica, which contains mutations of the sequence between -170 and -62.

The resulting plasmid, called pBB2, was then used to transform virulent cells of B. paratpertussis and B. bronchiseptica.

It was shown by analysis that the CAT activity was about 40% of the activity measured for plasmid pBP2.

This result agreed with those obtained by analysis of deletion of the terminal 5′ region.

According to the invention the modulating effect of MgSO$_4$ and temperature on the expression of proteins was tested by using cells of virulent B. bronchiseptica 7865 as reported in Example 5.

Also, in order to check the efficiency of the induction system according to the invention in a host other than Bordetella, the plasmid pLA - 14 - vir was constructed, and cells of E. coli can be transformed therewith as reported in Example 6.

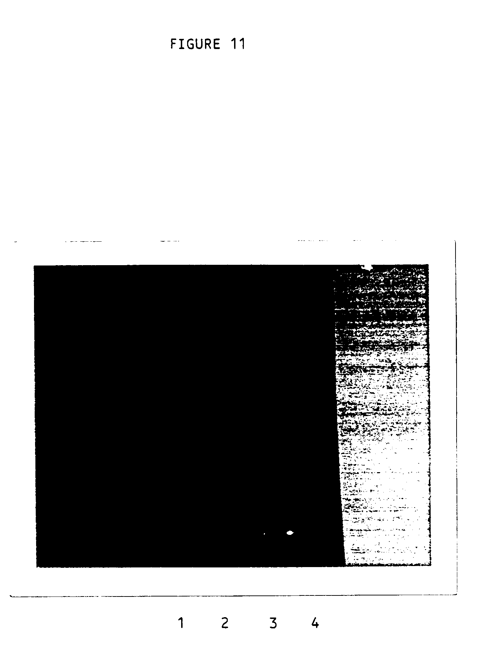

The results (Fig. 11) show that this inducible system is also active in E. coli.

This induction system therefore appears particularly suited for construction of vectors of use for producing high yields of heterologous proteins.

Brief description of the drawings

Fig. 1: Cloning of various fragments of the promoter PT in front of the CAT gene in the vector pLAFR2.

Fig. 2: Thin-layer chromatography of analysis of CAT from strains of Bordetella vir$^+$ (BP 356) and vir$^-$ - (BP 347) containing pBP1 and pBP2 recombinant plasmids respectively.

To display the results more clearly, the analysis was carried out on dilute extracts. Bands: A, chloramphenicol; B, C, chloramphenicol monoacetylate; D, chloramphenicol diacetylate.

Fig. 3: Comparison between the nucleotide sequences of the promoter regions of PT from B. pertussis 165 (BP), B. parapertussis 9305 (BPP) and B. bronchiseptica 4617 (BB). The bases, which are different in BPP and BB, are shown under the BP sequence. The -35 and -10 regions of the PT promoters are shown in boxes. The initial translation site, indicated by an arrow, is marked +1 and the other nucleotides are numbered in relation thereto. The positions of the various deletions are shown in the upper part of the sequence and indicated by a Δ followed by the clone number.

The diagram also shows the restriction sites used in the cloning procedures.

The bases derived from the polylinkers of vector pEMBL18 are written in small letters.

The arrows in front of the sequence indicate the repeating regions.

Fig. 4: CAT activity of various deletion mutants (expressed as % acetylation) in relation to the length of the terminal 5′ region of PT.

The dark columns show the CAT activity in strain BP356 vir$^+$ whereas the white columns show activity in strain BP347 vir$^-$. The chain lines define four levels of CAT activity chosen to illustrate the discussion. The standard deviations are between 10 and 7% at level A, between 7 and 5% at level B and below 5% in level C.

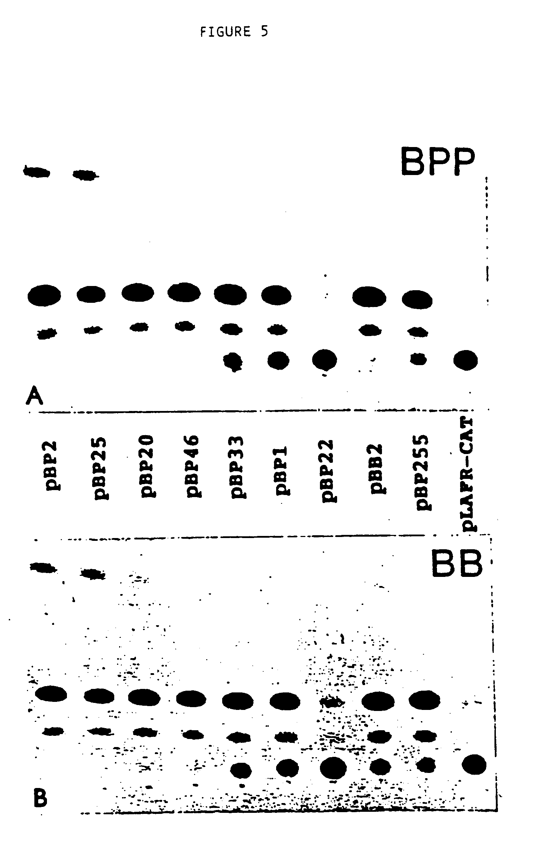

Fig. 5: Thin-layer chromatograph of analysis of CAT with various mutants of the promoter region of PT in B. parapertussis P14 (A) and in B. bronchiseptica 7865 (B).

In order to display the results more clearly, the analysis was carried out on non-dilute extracts. The various forms of chloramphenicol are the same as in Fig. 2.

Fig. 6: Comparison between the repeating sequences upstream of the promoter region. The bars indicate homologous nucleotides.

Fig. 7: Structure of the PT operon showing the distribution of mutations common to BPP and BB (vertical bars in the sequence given hereinbefore).

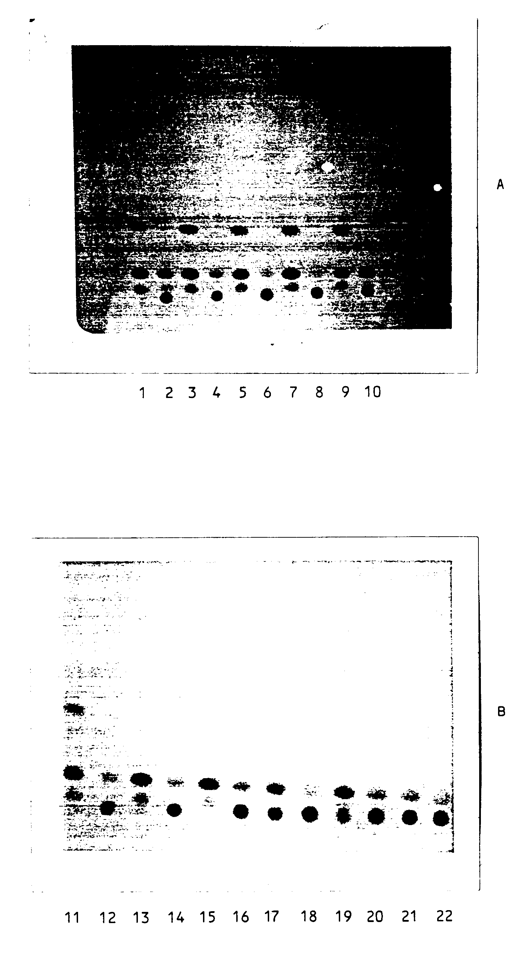

Figs. 8A and 8B: Modulation with MgSO₄ in virulent B. bronchiseptica transformed by pBP plasmids containing the various deletions in the terminal 5′ region of the PT operon :

```
1    PBP2 -;    3 ⎫ pBP14 -;    5 ⎫ pBP25 -;    7 ⎫pBP20 -;
2         +;    4 ⎭        +;    6 ⎭        +;    8 ⎭       +;
9 ⎫ pBP24 -;  11 ⎫ pBP46  -;  13 ⎫ pBP28 -;  15 ⎫pBP72 -;
10 ⎭      +;  12 ⎭        +;  14 ⎭        +;  16 ⎭       +;
17 ⎫ pBP33 -;  19 ⎫ pBP1   -;  21 ⎫ pBP22 -;
18 ⎭      +;  20 ⎭        +;  22 ⎭        +;
```

+ and - denote the presence or absence of MgSO₄ in the culture medium.

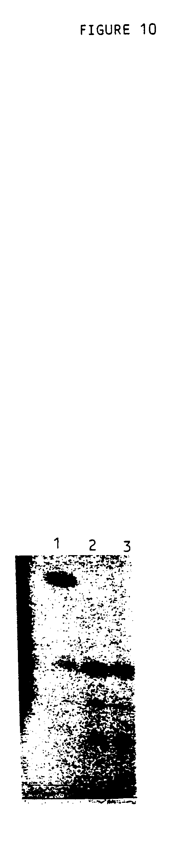

Fig. 9:
Identification of RNA by analysis of extension of the primer.
1) Reference sequence
2) pBP2 grown in absence of MgSO₄ at 35°C
3) pBP2 grown in presence of MgSO₄ at 35°C
4) pBP2 grown in absence of MgSO₄ at 25°C
5) pBP2 grown in absence of MgSO₄ at 35°C
Fig. 10: Modulation with temperature in B. bronchiseptica transformed with pBP2.
1) Growth in absence of MgSO₄ at 35°C
2) Growth in presence of MgSO₄ at 35°C
3) Growth in absence of MgSO₄ at 25°C.
Fig. 11: Expression and modulation of PT promoter in E. Coli. H1443, E. coli KY2562 ompR and E.coli FN 102 ompR transformed with pBP 14 and pLA-14 vir.

```
    ⎧ 1  pBP14  −                    ⎧ 5 pBP14  −
    ⎪ 2  pBP14  +                    ⎪ 6 pBP14  +
    ⎨                   H1443        ⎨                 K2562 ompR
    ⎪ 3 pLA-14-vir −                 ⎪ 7 pLA-14-vir⁻
    ⎩ 4 pLA-14-vir +                 ⎩ 8 pLA-14-vir⁺

    ⎧ 9 pBP14  −
    ⎪ 10 pBP14  +     FN102 ompR
    ⎨
    ⎪ 11 pLA-14-vir −
    ⎩ 12 pLA-14-vir+
```

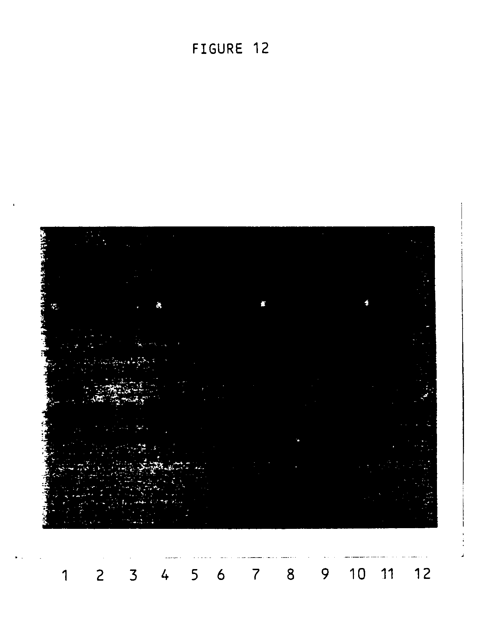

Fig. 12: Modulation with $MgSO_4$ in virulent and non-virulent B. bronchiseptica transformed with plasmid pFHA.

```
    1)  pBB 7865 (vir⁺)−        3   pBB (vir⁻) −
    2)                  +       4            +
```

+ and − indicate presence or absence of $MgSO_4$ in the culture medium.

Example 1

Construction of recombinant plasmids containing deletions in the 5′ region of the PT operon of B. pertussis.

A. Isolation of the structural gene which codes for CAT

20 $\mu$g of plasmid pA10 - CAT 2 (C. D'Onofrio et al (1985) J. EMBO 4, 1981-1989) were digested with 60 units (U) of each of the restriction enzymes HindIII and XbaI (Boehringer) in a buffer and under conditions suggested by the supplying firm.

After the enzymes had been inactivated at 65°C for 15 minutes, the digestion mixture was placed on 0.8% agarose gel and run at 100 V for 2 hours.

The 1636 bp HindIII - XbaI fragment containing the CAT gene without the promoter was then electro-eluted by the method of Maniatis (Molecular Cloning: a laboratory manual, Cold Spring Harbor, 1982).

B. Preparation of DNA fragments containing deletions in the terminal 5′ region upstream of the PT promoter

The deletions in the terminal 5′ region of the PT promoter were obtained by using Ba131 (BRL) exonuclease, following the method of R. J. Legerski et al (1978) (Nucl. Acids Res. 5, 1445).

In practice 10 $\mu$g of plasmid PT101 ATCC 67854 were digested with 30 U of EcoRI and HindIII (Boehringer).

After the enzymes had been inactivated at 65°C for 15 minutes, the digestion mixture was placed on 1.3% agarose gel and run at 100 V for 2 hours.

The 539 bp EcoRI-HindIII fragment containing the PT promoter with the 5′ region thereof was then electro-eluted from the gel. 200 ng of the fragment were then bonded to a bluescript SK vector (Stratagene, California) which had previously been digested with EcoRI and HindIII restriction enzymes by the method suggested by Boehringer, the supplying firm.

The ligation reaction was carried out in 30 $\mu$l of buffer solution (66 mM Tris-HCl pH 7.6, 1 mM ATP, 10 mM MgCl$_2$ and 20 mM dithiothreitol (DTT) in the presence of 1 U of T4 DNA ligase, at 14°C for 18 hours.

At the end, the entire mixture was used to transform cells of E.coli JM 101 (BRL) made competent with CaCl$_2$ by the method described by Messing (Methods in Enzymology Vol.101 20-78 (1983)).

The transformants were then selected on plates of LB agar (DIFCO) to which 100 $\mu$g/ml of ampicillin had been added, at 37°C for 18 hours.

The plasmid was isolated from one of the resulting positive clones, and had the expected characteristics.

40 $\mu$g of the plasmid were cut with 200 U of EcoRI at 37°C for 12 hours and then partially digested with 6 U of Ba131 (Boehringer) at 37°C, 2.5 $\mu$l portions being sampled every 30 seconds.

Next, 2 U of DNA polymerase (I) Klenow fragment was added to each thus-digested sample, in order to flatten the terminals, and was followed by 5 U of HindIII restriction enzyme. The mixtures were kept at 37°C for 5 hours and, after inactivation of the enzyme, were placed on 4% polyacrylamide gel and run at 250 V for 2 hours. DNA fragments containing 50 to 250 bases were then electro-eluted and cloned in bluescript SK vector, which had previously been digested with SmaI and HindIII enzymes, using the same method as before, and were sequenced as described by Sanger et al (1977) (PNAS USA 74, 5463).

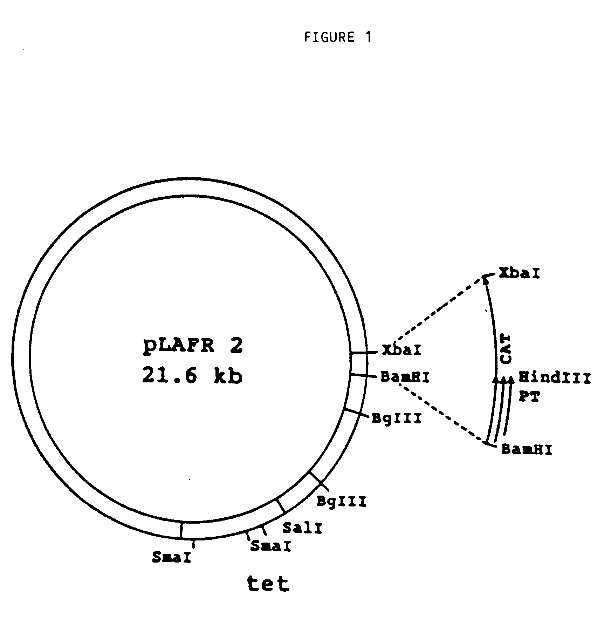

The BamHI-HindIII fragments with the deletions in the terminal 5′ region (Fig. 3) were isolated from the bluescript SK vector and cloned in plasmid pLAFR 2 (A. M. Friedman et al (1982) Gene 18, 289-296) (Fig. 1).

C) <u>Construction of hybrid plasmids with deletion in the terminal 5′ region of the PT promoter</u>

10 $\mu$g of plasmid pLAFR2 were digested with 50 U each of the enzymes BamHI and XbaI (Boehringer).

After inactivation of the enzymes, the mixture was placed on 0.5% agarose gel and run at 100 V for 2 hours. The 260 Kb bp fragment of DNA BamHI-XbaI was electro-eluted.

Next, 0.5 $\mu$g of BamHI-XbaI fragment and 0.3 $\mu$g of HindIII-XbaI fragment obtained as described in stage A) were added to 0.08 $\mu$g of each of the BamHI-HindIII fragments containing the various deletions and were ligated in 30 $\mu$l of ligase mixture in the presence of 2 U of T4 DNA ligase at 14°C overnight.

The ligase mixtures were used to transform competent cells of E.coli SM10 (R. Simon et al, (1983) Bio/Technology 1, 784-791) and the transformants were then selected at 37°C overnight on LB agar to which 12.5 $\mu$g/ml of tetracycline had been added.

Out of the resulting clones, those were isolated which contained recombinant plasmids having the expected characteristics.

The plasmids, containing the CAT gene in front of the PT promoter with the various deletions in its terminal 5′ region, were labelled pBP22, pBP1, pBP33, pBP72, pBP28, pBP46, pBP24, pBP20, pBP25, pBP14 and pBP2 (Fig. 4).

Fig. 3 gives the positions of the various deletions, indicated by $\Delta$, for each recombinant plasmid.

<u>Example 2</u>

<u>Description of the promoter region of the PT operon</u>

The hybrid plasmids obtained as described in Example 1 were introduced into a strain of B. pertussis BP356 (ptx:Tn5) Vir$^+$ and B. pertussis BP347 (Vir: Tn5) Vir$^-$ (A. A. Weiss et al (1983) Infec. Immun. 42, 33-41), using the conjugation technique.

In practice, the conjugations were brought about by using fresh cultures of the recipient species (Bordetella) and donator strains on Bordet-Gengou plates for at least six hours, following the method described by J. Bordet and O. Gengou (1909), Ann. Ins. Pasteur (Paris) 23, 415-419).

The exconjugates were then selected at 35°C for 15 hours on Bordet-Gengou plates to which 10 $\mu$g/ml tetracycline, 200 $\mu$g/ml streptomycin and 20 $\mu$g/ml of nalidixic acid had been added.

After being selected in this manner, the strains of Bordetella Vir$^+$ and Vir$^-$ were then grown in 10 ml of Stainer-Scholte medium (D. W. Stainer and M. J. Scholte (1971), J. Gen. Microbiol. 63, 211-220) containing 10 $\mu$g/ml of tetracycline at 35°C until the optical density measured at 580 nm was 0.8.

1.5 ml of each culture was then centrifuged at 5000 rpm for 5 minutes, using an Eppendorf centrifuge.

After being thus recovered, the cells were re-suspended in 300 $\mu$l of 0.25 M Tris-HCl buffer pH 7.8, broken by sonication in a Cell Disrupter (Bronson Sonie Parov Co) and re-centrifuged.

The supernatants were recovered (300 $\mu$l), incubated at 65°C for 10 minutes, centrifuged and then used to determine the presence and concentration of the resulting chloramphenicol acetyl transferase, by analysis of the activity thereof (A. Nicosia and R. Rappuoli (1987) J. Bacteriol. 169, 2843-2846; C. M. Gorman et al (1982) Mol. Cell. Biol. 2, 1044-1051).

In practice 15 $\mu$l of the supernatant were diluted to 150 $\mu$l with 0.25 M Tris-HCl buffer pH 7.8, and 1 $\mu$Ci of [$^{14}$C] chloramphenicol was added.

After incubation at 37°C for 5 minutes, 20 $\mu$l of 4 mM acetyl coenzyme A were added to each solution and the resulting mixtures were incubated at 37°C for 1 hour.

At the end of this period, the enzyme reaction was stopped by extracting the chloramphenicol and derivatives thereof with 2 ml of cold ethyl acetate (0°C).

The organic phases were removed by evaporation and the various forms of chloramphenicol were separated by thin-layer chromatography, using silica gel plates and a chloroform/methanol mixture (95 : 5, v/v) for one hour.

The plates were then placed in contact with a Kodak X-Omat AR X-ray plate at ambient temperature overnight, in order to display the results (Fig. 2).

For the purpose of quantitative determination of the expressed CAT, the samples were diluted from 1 : 10 to 1 : 300 and then analyzed as described hereinbefore.

After autoradiography the non-acetylated and the mono-acetylated forms of chloramphenicol were cut and counted. The conclusions from the results given in Figs. 2 and 4 are as follows:

- A region of at least -170 bp upstream of the initial transcription site, conventionally marked +1, is necessary for efficient activity of the promoter and consequently for transcription of the heterologous gene situated downstream of the aforementioned region;
- A vir region is necessary for the promoter region to be activated and the gene to be transcribed and
- Removal of the region between positions -182 and -171 results in a significant increase in expression of CAT. This is probably due to deletion of the palindromic sequence in the aforementioned region.

In order to confirm the results hereinbefore, we constructed two plasmids, pLAFR-CAT and pB255, for use as a negative and positive control respectively.

More particularly plasmid pLAFR-CAT was obtained by cloning the CAT gene in vector pLAFR2, digested with BamHI and XbaI restriction enzymes, the gene being without its own promoter, and using the BamHI-HindIII linker of pEMBL18 (L. Dente et al (1983) Nucl. Acids Res. 11, 1645-1655).

Plasmid pBP255, on the other hand, was constructed by insertion, upstream of the KpnI (-62) site (Fig. 3), of the 600 bp BamHI-HindIII fragment obtained from plasmid pT101ATCC 67854 (A. Nicosia et al (1987) Infect. Immun. 55, 963-967) which codes for the S1 sub-unit of PT deprived of the promoter.

The aforementioned plasmids were then introduced by conjugation into BP 356 (vir$^+$) and BP 347 (vir$^-$) and, after culture and lysis of the cells, the CAT activity was determined as described hereinbefore.

The results show that no CAT activity is present in either strain of Bordetella when transformed with plasmid pLAFR-CAT, whereas the strain BP356 (vir$^+$) containing pBP255 has the same activity as measured for plasmid pBP1.

This indicates that the specific sequences upstream of the PT promoter are necessary for transactivation thereof.

## Example 3

Cells of B. parapertussis P14 (SCLAVO) and B. Bronchiseptica 7865 (SCLAVO) in the virulent phase were conjugated with the plasmids containing the various deletions in the 5' region and with plasmid PLAFR-CAT.

The ex-conjugates were then cultivated in Stainer-Scholte medium as described in Example 2.

Finally, the cultures were analyzed to determine the expression of CAT, by determining the activity thereof.

The results, given in Fig. 5, appear identical with those obtained for B. pertussis BP356 (vir$^+$).

## Example 4

Following the procedure given in Example 3 hereinbefore, B. bronchiseptica 7865 in the virulent phase were conjugated with plasmid pBP2 obtained by replacing the region of pBP2 between the EcoRI (-483) and KpnI(-62) site by the corresponding region of B. bronchiseptica.

The last-mentioned region contains mutations in the sequence between -170 and -62, the mutations being also common to B. pertussis (Fig. 7).

The CAT activity, determined as described in Example 2, was about 40% of that determined for the same strain when transformed with pBP2, and was identical with that obtained for B. Bronchiseptica transformed with pBP46.

## Example 5

### Modulation of the vir region

Cells of virulent B. bronchiseptica 7865 were conjugated with plasmids pBP containing the various deletions in region 5′ of the PT operon .

The resulting ex-conjugates were separately inoculated in 150 ml Erlenmeyer flasks containing 10 ml of Stainer-Scholte medium and cultivated at 35°C overnight.

### A. The modulating effect of MgSO₄

10 μl of each pre-culture were then re-inoculated in duplicate in Erlenmeyer flasks, some of which contained 30 ml of Stainer-Scholte medium to which 70 mM $MgSO_4$ had been added, whereas others contained the medium alone.

The flasks were then kept at 35°C with gentle agitation until the optical density at 580 nm was 0.8.

Finally, 1.5 ml portions taken from each flask were analyzed to determine the expression of CAT by measuring the activity thereof. Simultaneously, 20-ml portions of each culture were analyzed to determine the presence or absence of RNA.

The RNA was quantitatively determined by the method of C. D'Onofrio et al (1985) EMBO I 4, 1981-1984).

The results, given in Fig. 8 (A and B) show the absence of CAT activity in the case of strains cultivated in the presence of $MgSO_4$. This indicates that this salt inhibits the regulating region.

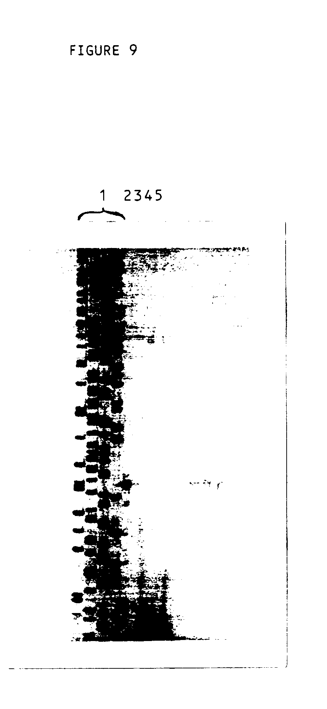

Also, the results in Fig. 9, which do not show any presence of RNA, confirm our previous observation, i.e. the absence of expression of the coding gene for CAT.

### B. Modulating effect of temperature

Cells of virulent B. bronchiseptica 7865 were conjugated with plasmid pBP2 and cultivated at 35°C overnight.

10μl portions of the preculture were used to inoculate Erlenmeyer flasks containing 30 ml of Stainer-Scholte medium.

Next, some flasks were cultivated at 35°C until the optical density at 580 nm was 0.8, whereas others were cultivated at ambient temperatures (20 - 25°C).

Finally, the individual cultures were analyzed to determine the CAT activity.

The results, given in Fig. 10, show absence of CAT activity in the case of the strains cultivated at ambient temperatures.

## Example 6

### Construction of plasmid pLA14-vir

### A. Isolation of the vir region from the chromosomal DNA of B. parapertussis

The strain B. parapertussis 9305 (Vir[+]) was cultivated in 500 ml of Stainer-Scholte medium at 37°C for 2 days.

Finally the cells were separated from the bouillon culture by centrifuging (10 minutes at 5000 rpm) in a Sorvall RC-5B centrifuge at 4°C, washed (2 x 120 ml) with a solution of 25% sucrose 100 mM NaCl and 50 mM Tris-HCl pH 7.5, and were recentrifuged as described hereinbefore.

The cells were resuspended in 10 ml of buffer solution (100 mM EDTA and 50 mM NaCl, pH 6.9) containing 1 mg/ml of lysozyme (Sigma).

The resulting suspension was then kept at 0°C for 30 minutes with gentle agitation.

Next, 1 ml of SDS (sodium dodecyl sulphate) was added to the suspension, followed after 10 minutes at 60°C by addition of 1 mg/ml of pronase pre-incubated at 37°C for 30 minutes in 1 x SSC (SSC = 1.15M NaCl, 15 mM sodium citrate). The resulting mixture was kept at 37°C for 2 hours.

After NaCl had been added to a final concentration of 1 M, the DNA was precipitated with 2 - 3 volumes of cold ethanol (-20°C), collected with a glass rod and resuspended in 10 ml of 0.1 x SSC.

The solution was kept at ambient temperature (20 - 25°C) with gentle agitation overnight and, after addition of RNAse (10γ/ml), at 37°C for 30 minutes.

The concentration of salt in the solution was then brought to 1 x SSC and, after extraction with phenol (1 volume), the DNA was precipitated by dropwise addition of isopropanol.

60 μg of the resulting chromosomal DNA were digested with 150 U of BamHI in 500 μl of reaction mixture for 12 hours.

The digestion mixture was then loaded on to a saccharose gradient (10 - 40%) in 35 ml of buffer comprising 1 mM NaCl, 10 mM Tris-HCl, pH 7.5 and 1 mM EDTA, and centrifuged in an SW28 Beckman rotor at 26000 rpm for 16 hours at 20°C.

Finally the gradient was divided into fractions and a portion of each fraction was analyzed on 0.7% agarose gel at 20 V overnight in order to identify the fractions containing fragments of approximately 14000 bp.

The fractions were then combined and the DNA was concentrated by precipitation with ethanol (-20°C).

After separation by centrifuging at 12000 rpm for 15 minutes, the DNA was cloned in plasmid pLAFR2 suitably digested with BamHI (Boehringer) (Fig. 1).

The resulting hybrid plasmids were conjugated in Bordetella pertussis vir⁺ and spread on Bordet-Gengou medium.

After one night at 35°C, those colonies were selected which showed a typical halo of vir⁺ strains.

Next, the plasmids were isolated from the aforementioned strains and digested with BamHI (Boehringer) restriction enzyme, using the same method as the supplying firm.

The fragment of approx. 4.7 Kb containing the vir region was isolated and ligated with plasmid pBP14 previously digested with BglIII Boehringer).

The ligation reaction was brought about as previously described, at 14°C for 18 hours.

The mixture was then used to transform competent cells of E.coli JM109, a microorganism obtainable from research centres.

The transformants were selected at 37°C overnight on LB agar (DIFCO) medium to which 12.5 μg/ml of tetracycline had been added. The plasmid having the expected characteristics was isolated from one of the positive clones and is hereinafter named pLA-14-vir.

### B. Expression and modulation in E.coli transformed with pLA-14-vir

Cells of E.coli H.1443, E.coli KY2562 ompR and E.coli FN102 ompR were transformed with plasmid pLA14-vir and pBP14 and cultivated at 37°C in 10 ml of LB medium to which 12.5 μg/ml of tetracycline had been added, until the optical density measured at 580 nm was 0.05.

Next, 5 μl of each culture were re-inoculated in duplicate in 10 ml of LB medium to which tetracycline (12.5 μg/ml had been added) and were cultivated at 35°C in the presence and absence of MgSO₄ (70 mM) until the optical density measured at 580 nm was 0.8.

Finally, 1.5 ml of each culture were examined to determine the CAT activity.

The results (Fig. 11) appear identical with those obtained for B. bronchiseptica conjugated with plasmid pBP 14. No difference was observed in the various strains of E.coli.

### Example 7

### Construction of plasmid pFHA

The chromosomal DNA from B. pertussis BP165 (SCLAVO) extracted and purified as described by Maniatis was digested with EcoRI and BamHI restriction enzymes (Boehringer). The digestion mixture was placed on 0.8% agarose gel and run at 100 V for 3 hours.

Finally, fragments containing from 4000 to 2000 bases were electro-eluted from the gel.

One of these fragments, made up of about 3500 nucleotides, appeared to comprise a non-coding region at 5′ and a region with a sequence corresponding to the aminoterminal sequence of FHA.

The fragment was cloned in vector pLARF2 in front of the structural CAT gene, using the polylinker of pUC18.

In practice the 3500 bp EcoRI/BamHI fragment, the pUC18 polylinker, the HindIII/XbaI fragment of 1636 bp containing the CAT gene and the plasmid pLARF2 digested with BamHI and XbaI, were bonded in the presence of T4 DNA ligase at 14°C overnight.

The ligation mixture was then used to transform cells of E.coli SM10 made competent, and the transformants were selected as described in Example 1.

The recombinant plasmids isolated from the positive clones were specified by means of a restriction map.

One of the plasmids, called pFHA, was conjugated in B. bronchiseptica vir$^+$ and vir$^-$, operating as described in Example 2.

The ex-conjugates were then cultivated in Stainer-Scholte medium in the presence and absence of MgSO$_4$, and at 35°C and at 25°C.

The CAT activity was then determined for the various cultures.

The results, given in Fig. 12, show that the modulating effect of MgSO$_4$ and temperature on the regulating system and on the expression of CAT is in the opposite direction from that observed when using the 5' region above the promotor PT of B. pertussis.

**Claims**

1. A novel system of inducible regulation of use for constructing a vector which can be replicated by expression, for use in the production of heterologous proteins by microbial expression, characterized in that it comprises the vir region of Bordetella which codes for one or more regulating factors, and also comprises a terminal 5' region situated upstream of a gene which codes for a Bordetella protein capable of being activated or inhibited in trans by the aforementioned factors and capable of enabling the promoter situated downstream thereof to control the expression of the protein of interest.

2. A novel regulating system according to claim 1, in which the terminal 5' region activated in trans by the regulating factor and/or factors is chosen from among those situated upstream of the gene (i) which codes for a virulent Bordetella protein.

3. A novel regulating system according to claim 2, in which the terminal 5' region is the 483 base pair sequence situated upstream of the recognition site of initial transcription of the operon which codes for the pertussis toxin produced by Bordetella pertussis.

4. A novel regulating system according to claim 3, in which the aforementioned sequence comprises at least 62 base pairs.

5. A novel regulating system according to claim 3, in which the sequence comprises 170 base pairs.

6. A novel regulating system according to claim 1, in which the terminal 5' region inhibited in trans by the regulating factor and/or factors is chosen from among those in front of the gene which codes for a non-virulent Bordetella protein.

7. A novel regulating system according to claim 6, in which the terminal 5' region is the non-coding sequence contained in the fragment of chromosomal DNA which codes for the aminoterminal part of the FHA protein.

8. A novel regulating system according to claim 1, in which induction is brought about by using temperatures of or about 35°C and a culture medium free from modulating substances such as MgSO$_4$, NaCl or nicotinic acid.

9. A novel regulating system according to claim 1, in which inhibition is brought about by using ambient temperatures (20 - 25°C) and a culture medium to which modulating substances such as MgSO$_4$, NaCl and nicotinic acid have been added.

10. A vector which can be replicated by expression and comprises a heterologous gene, expression of which is under the control of the positive regulating system according to claims 1 to 9.

11. A vector which can be replicated by expression according to claim 10, chosen from the group of plasmids of E.coli and Bacillus.

12. A microorganism transformed by a vector which can be replicated by expression according to claim 10, capable of producing a heterologous protein by expression of the coding gene by the aforementioned protein where the said expression is under the control of the regulating system according to claims 1 to

EP 0 336 413 B1

9.

**13.** A microorganism transformed according to claim 12 and chosen from the group comprising <u>Escherichia coli</u>, <u>Bordetella</u> and <u>Bacillus</u>.

**14.** A method of producing a heterologous protein in high yields, comprising cultivation in a suitable culture medium of a microorganism transformed according to claims 12 and 13.

**15.** A method according to claim 14 in which the transformed microorganism is cultivated in a culture medium in the presence or absence of modulating substances and at ambient temperatures (20 - 25°C) or temperatures equal to or approximately 35°C with the regulating system in inactivated form for sufficient time to obtain the maximum concentration of cells, the temperature and the culture medium being subsequently changed so that the resulting regulating system can transcribe the gene which codes for the protein of interest.

**16.** Use of the regulating system according to claim 1 for producing a vector which can be replicated by expression according to claim 10.

**17.** Use of the vector which can be replicated by expression according to claim 10 for obtaining a microorganism transformed according to claim 12.

**Patentansprüche**

**1.** Neues System einer induzierbaren Regulation zur Verwendung in der Konstruktion eines Vektors, der durch Expression repliziert werden kann, zur Verwendung in der Produktion von heterologen Proteinen durch mikrobielle Expression, dadurch gekennzeichnet, daß es die vir-Region von Bordetella umfaßt, die einen oder mehrere regulierende Faktoren codiert, und auch einen terminalen 5'-Bereich umfaßt, der stromaufwärts von einem Gen liegt, das ein Bordetella-Protein codiert, welches durch die vorstehenden Faktoren in trans aktiviert oder inhibiert werden kann und dem stromabwärts liegenden Promotor die Fähigkeit zur Kontrolle der Expression des Proteins von Interesse verleihen kann.

**2.** Neues regulierendes System nach Anspruch 1, wobei der terminale 5'-Bereich, der in trans durch den regulierenden Faktor und/oder die Faktoren aktiviert wird, ausgewählt ist aus denen, die stromaufwärts vom Gen (i) liegen, das ein virulentes Bordetella-Protein codiert.

**3.** Neues regulierendes System nach Anspruch 2, wobei der terminale 5'-Bereich die 483 Basenpaare umfassende Sequenz ist, die stromaufwärts von der Erkennungsstelle des Transkriptionsstarts des Operons liegt, das das Pertussistoxin codiert, welches von Bordetella pertussis produziert wird.

**4.** Neues regulierendes System nach Anspruch 3, wobei die vorstehend erwähnte Sequenz mindestens 62 Basenpaare umfaßt.

**5.** Neues regulierendes System nach Anspruch 3, wobei die Sequenz 170 Basenpaare umfaßt.

**6.** Neues regulierendes System nach Anspruch 1, wobei der terminale 5'-Bereich, der in trans durch den regulierenden Faktor und/oder Faktoren inhibiert wird, ausgewählt ist aus denen, die vor dem Gen liegen, welches ein nicht-virulentes Bordetella-Protein codiert.

**7.** Neues regulierendes System nach Anspruch 6, wobei der terminale 5'-Bereich die nicht-codierende Sequenz ist, die im Fragment der chromosomalen DNA enthalten ist, die den aminoterminalen Teil des FHA-Proteins codiert.

**8.** Neues regulierendes System nach Anspruch 1, wobei die Induktion durch Verwendung von Temperaturen von etwa 35°C und eines Kulturmediums erzielt wird, das frei ist von modulierenden Stoffen, wie $MgSO_4$, NaCl oder Nicotinsäure.

**9.** Neues regulierendes System nach Anspruch 1, wobei die Hemmung durch Verwendung von Raumtemperaturen (20 bis 25°C) und eines Kulturmediums erzielt wird, dem modulierende Substanzen, wie

14

MgSO$_4$, NaCl und Nicotinsäure, zugefügt wurden.

10. Vektor, der durch Expression repliziert werden kann und ein heterologes Gen umfaßt, dessen Expression unter der Kontrolle des positiven regulierenden Systems nach den Ansprüchen 1 bis 9 steht.

11. Vektor, der durch Expression nach Anspruch 10 repliziert werden kann, ausgewählt aus Plasmiden von E. coli und Bacillus.

12. Mikroorganismus, der durch einen Vektor transformiert ist, der durch Expression nach Anspruch 10 repliziert werden kann, der fähig ist zur Produktion eines heterologen Proteins durch Expression des für das vorstehend erwähnte Protein codierenden Gens, wobei die Expression unter der Kontrolle des regulierenden Systems nach den Ansprüche 1 bis 9 steht.

13. Mikroorganismus, der nach Anspruch 12 transformiert ist und ausgewählt ist aus Escherichia coli, Bordetella und Bacillus.

14. Verfahren zur Herstellung eines heterologen Proteins in hohen Ausbeuten, umfassend die Züchtung eines gemäß den Ansprüchen 12 und 13 transformierten Mikroorganismus in einem geeigneten Kulturmedium.

15. Verfahren nach Anspruch 14, wobei der transformierte Mikroorganismus in einem Kulturmedium in Gegenwart oder Abwesenheit von modulierenden Stoffen und bei Umgebungstemperaturen (20 bis 25°C) oder Temperaturen, die ungefähr oder genau bei 35°C liegen, mit dem regulierenden System in inaktivierter Form für einen ausreichend langen Zeitraum gezüchtet wird, um die maximale Konzentration an Zellen zu erhalten, wobei die Temperatur und das Kulturmedium anschließend geändert werden, so daß das erhaltene regulierende System das Gen transkribieren kann, welches das Protein von Interesse codiert.

16. Verwendung des regulierenden Systems nach Anspruch 1 zur Herstellung eines Vektors, der durch Expression nach Anspruch 10 repliziert werden kann.

17. Verwendung des Vektors, der durch Expression nach Anspruch 10 repliziert werden kann, zur Gewinnung eines Mikroorganismus, der gemäß Anspruch 12 transformiert wurde.

**Revendications**

1. Nouveau système de régulation inductible utilisable pour la construction d'un vecteur pouvant être répliqué par expression, pour utilisation dans la production de protéines hétérologues par expression microbienne, caractérisé en ce qu'il comprend la région vir de Bordetella qui code pour un ou plusieurs facteurs de régulation, et comprend également une région terminale en 5' située en amont d'un gène qui code pour une protéine de Bordetella capable d'être activée ou inhibée en trans par les facteurs précités, et qui est capable de permettre au promoteur situé en aval de celui-ci de réguler l'expression de la protéine d'intérêt.

2. Nouveau système de régulation selon la revendication 1, dans lequel la région terminale en 5', activée en trans par le facteur et/ou les facteurs de régulation, est choisie parmi celles situées en amont du gène (i) qui code pour une protéine d'une souche virulente de Bordetella.

3. Nouveau système de régulation selon la revendication 2, dans lequel la région terminale en 5' est la séquence de 483 paires de bases située en amont du site de reconnaissance d'initiation de transcription de l'opéron qui code pour la toxine de la coqueluche produite par Bordetella pertussis.

4. Nouveau système de régulation selon la revendication 3, dans lequel la séquence précitée comprend au moins 62 paires de bases.

5. Nouveau système de régulation selon la revendication 3, dans lequel la séquence comprend 170 paires de bases.

6. Nouveau système de régulation selon la revendication 1, dans lequel la région terminale en 5' qui est inhibée en trans par le facteur et/ou les facteurs de régulation est choisie parmi celles en amont du gène qui code pour une protéine d'une souche non virulente de Bordetella.

7. Nouveau système de régulation selon la revendication 6, dans lequel la région terminale en 5' est la séquence non codante contenue dans le fragment d'ADN chromosomique qui code pour la partie amino-terminale de la protéine FHA.

8. Nouveau système de régulation selon la revendication 1, dans lequel l'induction est réalisée au moyen de températures de ou d'environ 35°C et d'un milieu de culture exempt de substances modulatrices telles que $MgSO_4$, NaCl ou l'acide nicotinique.

9. Nouveau système de régulation selon la revendication 1, dans lequel l'inhibition est réalisée au moyen de températures ambiantes (20-25°C) et d'un milieu de culture auquel ont été ajoutées des subtances modulatrices telles que $MgSO_4$, NaCl et l'acide nicotinique.

10. Vecteur qui peut être répliqué par expression et comprend un gène hérérologue, dont l'expression est sous le contrôle du système de régulation positive selon les revendications 1 à 9.

11. Vecteur qui peut être répliqué par expression selon la revendication 10, choisi dans le groupe des plasmides de E. coli et Bacillus.

12. Micro-organisme transformé par un vecteur qui peut être répliqué par expression selon la revendication 10, capable de produire une protéine hétérologue par expression du gène codant pour la protéine précitée, ladite expression étant sous le contrôle du système de régulation selon les revendications 1 à 9.

13. Micro-organisme transformé selon la revendication 12 et choisi dans le groupe comprenant Escherichia coli, Bordetella et Bacillus.

14. Procédé de production d'une protéine hétérologue avec de hauts rendements, comprenant la culture, dans un milieu de culture approprié, d'un micro-organisme transformé selon les revendications 12 et 13.

15. Procédé selon la revendication 14, dans lequel le micro-organisme transformé est cultivé dans un milieu de culture en présence de substances modulatrices et aux températures ambiantes (20-25°C) ou à des températures égales à ou d'environ 35°C, avec le système de régulation sous une forme inactivée, pendant une durée suffisante pour l'obtention de la concentration maximale de cellules, la température et le milieu de culture étant ensuite modifiés de manière que le système de régulation résultant puisse transcrire le gène qui code pour la protéine d'intérêt.

16. Utilisation du système de régulation selon la revendication 1, pour la production d'un vecteur qui peut être répliqué par expression selon la revendication 10.

17. Utilisation du vecteur qui peut être répliqué par expression selon la revendication 10, pour l'obtention d'un micro-organisme transformé selon la revendication 12.

FIGURE 1

FIGURE 2

FIGURE 3

```
              Δ2
             -483                                         -433        -200
BP   gatcctctagagtGAATTCGTCGCCTCGCCCTGGTTCGCCGTCATGGCCCCCAAGG.......ACCATCCC
BPP
BB   BamHI        EcoRI


     Δ14        Δ25           Δ20      Δ24                    Δ46     Δ28
    -183       -171          -158     -149                   -130    -123
BP   GCATACGTGTTGGCAACCGCCAACGCGCATGCGTGCAGATTCGTCGTACAAAACCCTCGATTCTTCCGTACA
BPP                          T       C G   GCGGATC      G         A C       C
BB                          T       C G         C      G                   C


              Δ72            Δ33              Δ1
             -97            -80             -62
BP   TCCCGCTACTGCAATCCAACACGGCATGAACGCTCCTTCGGCGCAAAGTCGCGCGATGGTACCGGTCACCGT
BPP                T        GCA       C              A      KpnI          G
BB                          GC                       A


              Δ22                                   mRNA
             -37                      -10
BP   CCGGACCGTGCTGACCCCCCTGCCATGGTGTGATCCGTAAAATAGGCACCATCgacctgcaggcatgcaagc
BPP              C                    C          G   CA
BB   A    T     C                    C          G   C                 HindIII
```

FIGURE 4

FIGURE 5

FIGURE 6

```
-157   G A T T C G T C . G T A C A A A A C C C T C   -137
       | | | | | | |   | | | | | |       | | | |
-136   G A T T C T T C C G T A C A . T . C C C G C   -117
```

FIGURE 7

FIGURE 8

1 2 3 4 5 6 7 8 9 10

A

11 12 13 14 15 16 17 18 19 20 21 22

B

FIGURE 9

FIGURE 10

FIGURE 11

1    2    3    4

FIGURE 12

1 2 3 4 5 6 7 8 9 10 11 12